# EUROPEAN PATENT SPECIFICATION

(11) **EP 0 813 597 B1**
(45) Date of publication and mention of the grant of the patent: **04.10.2006**
(21) Application number: 96904055.9
(22) Date of filing: 10.02.1996
(51) Int. Cl.: C12N 9/64, C07K 14/745, B01D 15/08, B01J 20/32

(54) **A PROCESS FOR THE PURIFICATION OF VITAMIN K DEPENDENT COAGULATION FACTORS BY CHROMATOGRAPHY**
PROZESS ZUR REINIGUNG VON VITAMIN K ABHÄNGIGEN KOAGULATIONSFAKTOREN DURCH CHROMATOGRAPHIE
PROCEDE DE PURIFICATION PAR CHROMATOGRAPHIE DE FACTEURS DE COAGULATION DEPENDANTS DE LA VITAMINE K

(30) Priority: 15.02.1995 DE 19504852
(43) Date of publication of application: 29.12.1997
(73) Proprietor: Octapharma AG, 8853 Lachen (CH)
(72) Inventor: JOSIC, Djuro COBRE CCRD, The CORO Center, RI 02903, Providence (US); MORFELD, Frank, D-67560 Eschborn (DE)
(74) Representative: Meyers, Hans-Wilhelm
(86) International application number: PCT/EP1996/000569
(87) International publication number: WO 1996/025490

(56) References cited:
- EP-A- 0 317 376
- EP-A- 0 354 354
- EP-A- 0 396 022
- EP-A- 0 617 049
- "Biochemica catalogue 92/93; pages 187-188" , MERCK , DARMSTADT (GER) XP002005741 * p.187, Fractogel EMD; p. 188, Fractogel TSK AF- Amino *
- ANN. HEMATOL., vol. 70 , no. suppl. 1, page a84 XP000570906 MORFELD, F. ET AL. : "Vitamin K dependent clotting factors: ..."
- BURNOUF ET AL VOX SANG vol. 57, 1989, pages 225 - 232
- FELDMAN ET AL BLOOD COAGULATION AND FIBRINOLYSIS vol. 5, 1944, pages 939 - 948
- Phd-thesis od Dr. Kraus (pages 41-42).

## Description

The present invention pertains to a process for the purification of vitamin K dependent coagulation factors by anion-exchange chromatography and to a PPSB fraction or a proteins C and S as well as factor IX fraction obtainable by the process according to the invention, as well as a chromatographic material.

EP 0 396 022 describes the preparation of a factor IX concentrate from plasma by chromatography, namely affinity chromatography by means of a carrier material in which 2-hydroxyaminopropyl (HAP) groups are bonded to a relatively short chain of 3 carbon atoms (spacer). Following a heparin affinity chromatographic step, a factor IX concentrate can be prepared by the method described therein which is said to contain a specific factor IX activity of above 200 U/mg. The overall yield of the factor, based on plasma, is said to be near 30% when washing is performed with aqueous solutions containing only NaCl as a salt (for increasing conductivity). 1-Amino-2-propanol serves as the eluent. This is a drawback for drug safety reasons. In addition, the yield will decrease according to the procedure described in EP 0 396 022 when virus inactivation steps are performed. This is absolutely required, however, when pooled plasma is used in order to prevent viral transmission with the vitamin K dependent factors (e.g. hepatitis A and B, HIV, etc.).

Although the presently known anion exchange or affinity chromatographic materials are suitable to ensure chromatographic separation of the vitamin K dependent coagulation factors, it is desirable to provide chromatographic materials which enable further improvement of the separation of vitamin K dependent coagulation factors from blood plasma.

Thus, the object of the present invention is, on one hand, to provide a process which enables an improved yield of vitamin K dependent coagulation factors, such as PPSB concentrates, proteins C and S/factor IX fractions, and to provide a chromatographic material which is particularly useful for preparing vitamin K dependent coagulation factors from appropriate sources.

The object of the present invention is achieved by a process according to claim 1. Claims 2 to 10 pertain to preferred embodiments of the process according to the invention. Claims 11 and 12 are directed to products containing vitamin K dependent coagulation factors which are characterized by a high purity and high specific activity and obtainable by the process according to the invention. Claim 13 is concerned with a protein C and S containing fraction obtainable by step c) of claim 1. Claim 14 pertains to the chromatographic material which may be used in the process according to the invention.

According to the invention, there is added to the source containing the vitamin K dependent factors a material to which HAP ligands are bonded through so-called tentacles. The term "tentacles" refers to certain spacers with which chromatographic material is modified as described in EP 0 337 144. The chromatographic material modified with HAP ligands to which the ligand is bonded through tentacles exhibits novel properties resulting in the object of the invention's being achieved. The HAP ligands covalently bonded to the chromatographic material through the tentacles are bonded to the matrix in a freely movable manner through the tentacles. By using this material, it is possible to perform chromatography of the vitamin K dependent coagulation factors from undiluted plasma and to recover the vitamin K dependent coagulation factors in high yield and purity. The fractions of the coagulation factors recovered according to the invention together will then give highly pure coagulation factor concentrates following the purification steps of the process according to the invention.

The figure shows a flow-chart of the procedure of the process according to the invention with preferred embodiments for preparing a PPSB concentrate and a factor IX concentrate.

According to the invention, a source containing vitamin K dependent factors is separated into the desired fractions by means of affinity chromatography. This is done on a material modified with 2-hydroxyaminopropyl groups. Particularly preferred is a material obtainable by coupling 2-hydroxyaminopropyl groups to a matrix resulting in the so-called tentacle chromatographic materials (Merck). The modification of the material and the material itself will be characterized below.

The source containing vitamin K dependent factors is contacted with the affinity material from a solution, this solution having a relatively low ionic strength, corresponding to from 10 to 150 mOsm. This is achieved in a particularly simple way by charging the chromatographic material, preferably as a slurry, into a chromatographic column and charging the solution with the source containing vitamin K dependent factors on the column. The ionic strength is adjusted such that the vitamin K dependent coagulation factors remain adsorbed to the chromatographic material whereas other components are washed from the chromatographic column. Optionally, following application of the solution containing the vitamin K dependent coagulation factors, washing may be performed with a solution of similar ionic strength as that used in the application. Then, according to step b) of the process according to the invention, a solution with higher ionic strength is contacted with the chromatographic material. This will result in desorption of the vitamin K dependent coagulation factors adsorbed to the chromatographic material according to the invention from the surface of the chromatographic material and elution from the chromatographic column. This fraction contains the PPSB factors IX, II, X, and VII, and the proteins C and S.

Then, this fraction can be further processed into a commercial PPSB preparation in a per se known manner.

For isolation of factor IX, the fraction eluting according to step b) is collected and subjected to a second chromatography in the form of a heparin affinity chromatography. Heparin affinity chromatography is known as such and described in more detail in D. Josic, F. Bal and H. Schwinn, Journal of Chromatography, 632 (1993), 1-10.

Finally, in order to obtain a preparation which is unobjectionable by current standards, a virus inactivation step is performed, preferably between steps b) and c) of the process according to the invention. Preferably, virus inactivation is carried out by chemical treatment with detergents and/or heat treatment. As detergents, non-ionic detergents may be used. These are combined with dialkyl/trialkyl substituted phosphates, such as TNBP (tri-n-butylphosphate), for instance. The detergents employed for virus inactivation can be used at up to 20%. The heat treatment step corresponds to a pasteurizing step, preferably in a temperature range of from 60 to 65°C for 5 to 30 hours, especially for 8 to 14 hours (WO 94/17834).

In a further embodiment of the process according to the invention, step c) may be followed by a second virus inactivation step. This is advisable especially when only one virus inactivation is performed between steps b) and c). Particularly preferred is chemical virus inactivation between steps b) and c), and physical virus inactivation, for example, heat treatment, following step c) of the process according to the invention.

If a second virus inactivation step is performed following step c) of the process according to the invention, a third chromatographic purification operation is recommended which is one of a second heparin affinity chromatography, anion-exchange chromatography, or chromatography on the carrier material used in step a) of the process according to the invention.

In another embodiment of the process according to the invention, physical virus inactivation, for example, heat treatment, may be performed following step b). Preferably, this is followed by an intermediate purification which is either anion-exchange chromatography or another chromatography on the carrier material according to the invention used in step a) of the process according to the invention. This may be followed by solvent/detergent inactivation (EP 131 740 A2) and heparin affinity chromatography.

As the source containing vitamin K dependent coagulation factors, there may be used, in particular, blood plasma or cryopoor plasma or cell culture supernatants or other solutions containing vitamin K dependent coagulation factors.

Depending on the condition of the source containing vitamin K dependent coagulation factors, filtration through biocompatible materials may be recommended. As filters, there may be used, in particular, filters having pore sizes of between 0.5 and 2.0 µm.

By means of the process according to the invention, concentrates may be prepared containing vitamin K dependent coagulation factors. The factor IX and PPSB concentrates have higher purity as compared to the prior art concentrates and are therefore novel substances. Further, the proteins C and S have a specific activity of from 8 to 10 U/mg in the PPSB concentrate. Thus, a useful starting material is obtained for the preparation of a new high purity concentrate of proteins C and S.

The chromatographic material which may be employed in the process according to the invention can be obtained by modification of so-called tentacle materials with 2-hydroxyaminopropyl groups (HAP) as ligands. The chromatographic material according to the invention has a hydrophilic moveable arm (spacer) on the base matrix, or carrier material. To this arm the HAP group is bonded. Surprisingly, it is shown that by using the chromatographic material according to the invention, purification of the vitamin K dependent coagulation factors from appropriate sources is possible with high purity and yield.

The invention will be illustrated in more detail by the following examples.

Examples 1 and 2 include a comparison between the chromatographic material described in EP 0 396 022 and the chromatographic material according to the invention under otherwise authentic conditions mimicking those used in EP 0 396 022. Example 1 includes the use of the material according to the invention while Example 2 includes the use of the material described in EP 0 396 022.

### Example 1

A 10 ml column filled with a matrix having 2-hydroxyaminopropyl ligand bonded thereto through a tentacle arm is equilibrated with 60 ml of buffer A (10 mM citrate, 10 mM NaHPO₄, 100 mM NaCl, pH 7.5).

Two hundred ml of cryopoor plasma is applied at a linear flow rate of 100 cm/h and then washed with 50 ml of buffer A. Subsequently, factor IX is eluted with buffer A containing 0.8 M NaCl. 30% of factor IX is found in the eluate while 50% is eluted with 0.8 M NaCl. The specific activity of factor IX is 6.5 U/mg. The enrichment in the 0.8 M NaCl eluate of protein C is 30% and of protein S 20% of the initial amount.

### Example 2

A 10 ml column filled with a matrix bearing 2-hydroxyaminopropyl ligand consisting of a copolymer of glycidyl methacrylate, pentaerythritol dimethacrylate, and poly(vinyl alcohol) (Toyopearl TSK-Amino, TosoHaas) is equilibrated with 60 ml of buffer A (10 mM citrate, 10 mM NaHPO₄, 100 mM NaCl, pH 7.5).

Two hundred ml of cryopoor plasma is applied at a linear flow rate of 100 cm/h and then washed with 50 ml of buffer A. Subsequently, factor IX is eluted with buffer A containing 0.5 M NaCl. Seventy-five % of factor IX is found in the eluate while 13% is eluted with 0.5 M NaCl. The specific activity of factor IX is 7 U/mg. The enrichment of protein C in the 0.5 M NaCl eluate is only 2% of the initial amount while protein S is not detectable.

Example 3 shows the result obtained by using the material according to the invention in the process according to the invention under different buffer and elution conditions, Example 3 showing an optimization according to the invention, and Example 4 showing the result which can be achieved with the conventional material using the optimized conditions according to the invention.

### Example 3

A 10 ml column filled with a matrix having 2-hydroxyaminopropyl ligand bonded thereto through a tentacle arm is equilibrated with 60 ml of buffer A (20 mM citrate, pH 7.5).

Two hundred ml of cryopoor plasma is applied at a linear flow rate of 100 cm/h and then washed with 50 ml of buffer A -containing 0.15 M NaCl. Subsequently, factor IX is eluted with buffer A containing 0.8 M NaCl. Eighteen % of factor IX is found in the eluate while 75% is eluted with 0.8 M NaCl. The specific activity of factor IX is 7 U/mg. The enrichment in the 0.8 M NaCl eluate of protein C is 50% and of protein S 30% of the initial amount.

### Example 4

A 10 ml column filled with a matrix bearing 2-hydroxyaminopropyl ligand consisting of a copolymer of glycidyl methacrylate, pentaerythritol dimethacrylate, and poly(vinyl alcohol) (Toyopearl TSK-Amino, TosoHaas) is equilibrated with 60 ml of buffer A (20 mM citrate, pH 7.5) .

Two hundred ml of cryopoor plasma is applied at a linear flow rate of 100 cm/h and then washed with 50 ml of buffer A. Subsequently, factor IX is eluted with buffer A containing 0.5 M NaCl. Sixty-one % of factor IX is found in the eluate while 32% is eluted with 0.5 M NaCl. The specific activity of factor IX is 6 U/mg. The enrichment in the 0.5 M NaCl eluate of protein C is 5% and of protein S 1% of the initial amount.

A comparison of Examples 3 and 4 shows that surprisingly a double recovery of factor IX can be achieved by using the material according to the invention. At the same time, and in contrast to the prior art process, proteins C and S are also enriched in this fraction.

In Example 5, another optimization with the material according to the invention is shown. In Example 6, the process has been performed under the application and washing conditions according to the invention but using a prior art carrier material.

### Example 5

A 250 ml column filled with a matrix having 2-hydroxyaminopropyl ligand bonded thereto through a tentacle arm is equilibrated with 2000 ml of buffer A (20 mM citrate, pH 7.5).

Five thousand ml of cryopoor plasma is applied and washed with 750 ml of buffer A. Then, washing is performed with 2000 ml of buffer A containing 0.15 M NaCl. Subsequently, factor IX is eluted with buffer A containing 0.8 M NaCl, 0.1% of amino acids, such as lysine, 0.1% of arginine. Factor IX of this fraction has a specific activity of 8 U/mg. The recovery of factor IX is 85% of the initial activity in the cryopoor plasma. In this fraction, protein C is contained at 55% and protein S at 40% of the initial amount.

The eluate is diafiltrated against 20 mM citrate buffer, pH 7.0, until an osmolality of 200 mOsmol is achieved. To the solution is added 1% of Tween and 0.3% of TNBP followed by incubation at 25°C for 7 hours.

A 500 ml Heparin-Sepharose (Pharmacia) is equilibrated with 1500 ml of buffer A. The factor IX eluate is applied and washed with 1000 ml of buffer A. Subsequently, factor IX is eluted with a linear gradient of 0-1 M NaCl in buffer A. The fractions with a specific activity of factor IX of more than 200 are pooled, stabilizers are added, and pasteurization is performed. Then, the diluted solution is subjected to a second heparin chromatography. A doubly virus inactivated factor IX concentrate is obtained with a specific activity of >200 U/mg with a recovery of 40% from plasma.

### Example 6

A 10 ml column filled with a matrix bearing 2-hydroxyaminopropyl ligand consisting of a copolymer of glycidyl methacrylate, pentaerythritol dimethacrylate, and poly(vinyl alcohol) (Toyopearl TSK-Amino, TosoHaas) is equilibrated with 60 ml of buffer A (20 mM citrate, pH 7.5).

Two hundred ml of cryopoor plasma is applied at a linear flow rate of 100 cm/h and then washed with 50 ml of buffer A containing 0.15 M NaCl. Subsequently, factor IX is eluted with buffer A containing 0.5 M NaCl. Fifty % of the amount of factor IX is found in the eluate. Another 38% are found in the 0.5 M NaCl wash. Only 5% recovery of factor IX could be found in the 0.5 M NaCl eluate.

Example 6 makes clear that the use of the conventional material gives rise to essential drawbacks since the major portion of factor IX is in the eluate and in the washing fraction. Factor IX as well as the other vitamin K dependent coagulation factors will bind but insufficiently to the prior art chromatographic material when to be purified from viscous solutions, such as undiluted plasma, or from solutions with high ionic strength.

Example 7 includes the preparation of a PPSB concentrate using the chromatographic material according to the invention. Chromatography of the cryopoor plasma with Q-Sepharose Fast Flow was performed as a so-called capture step. This intermediate product was then subjected to chromatography using the chromatographic material according to the invention. The capture step can also be performed employing other gel materials highly exhibiting anion-exchange properties.

### Example 7

A 200 ml column filled with Q Sepharose FF (Pharmacia) is equilibrated with buffer A (0.005 M NaHPO₄, pH 6).

Five thousand ml of cryopoor plasma is applied at a flow rate of 100 cm/h and washed with 500 ml of buffer A. Then, the column is washed with 500 ml of buffer A containing 0.05 M NaCl. Subsequently, elution is performed with buffer A containing 0.4 M NaCl. This fraction is dialysed against 25 mM citrate, pH 7, until an osmolality of 150 mOsm is achieved. To the solution is added 1% of Tween and 0.3% of TNBP followed by incubation at 25°C for 8 hours.

A 50 ml column filled with a matrix having 2-hydroxyaminopropyl ligand bonded thereto through a tentacle arm is equilibrated with 2000 ml of buffer B (20 mM citrate, pH 7.5). The Q Sepharose eluate is applied on the column and washed with 300 ml of buffer B. Subsequently, washing with 250 ml of buffer B containing 0.1 M NaCl and then elution with buffer B containing 0.8 M NaCl, 0.1% of lysine, 0.1% of arginine is performed. A fraction is obtained containing the PPSB factors IX, II, X, and VII at a specific activity of 7 U/mg. The overall recovery of the PPSB factors is 54%. In addition, this fraction contains proteins C and S with a recovery of 40% and 35%, respectively.

Surprisingly, the process according to the invention and the material according to the invention results in isolation of factor IX along with proteins C and S, which may be directly from plasma. With the prior art HAP materials, however, the major fraction of proteins C and S is found in the eluate.

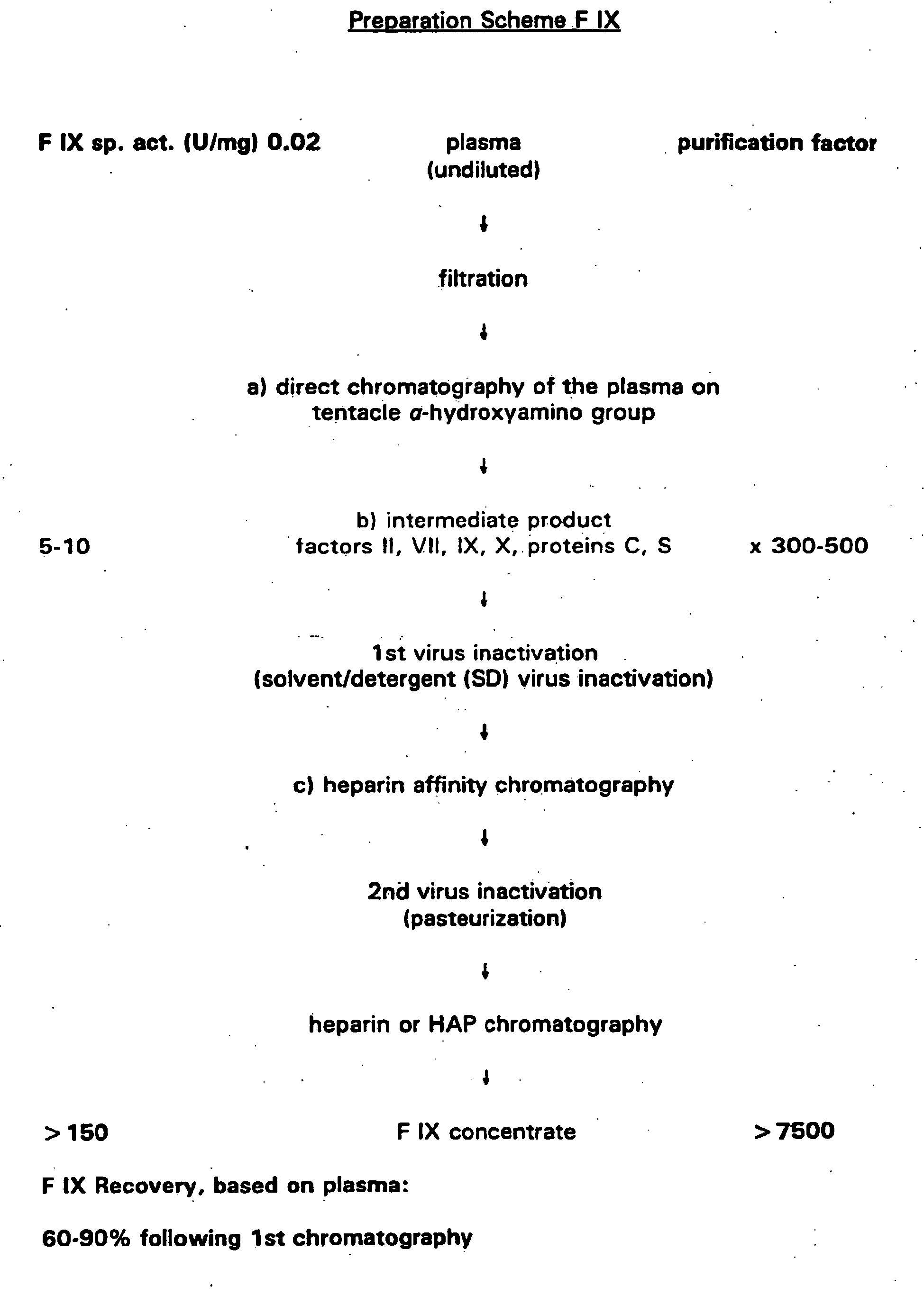

## Claims

1. A process for the purification of vitamin K dependent coagulation factors by affinity chromatography wherein fractions of a source containing said vitamin K dependent factors are
a) subjected to chromatography on a material modified with 2-hydroxyaminopropyl groups bonded to spacers having more than 3 carbon atoms, said source being applied from a solution whose ionic strength is chosen such that said vitamin K dependent coagulation factors are adsorbed on the above anion-exchange material, washing is optionally performed with an appropriate buffer solution, then, after separation of the eluate,
b) a buffer solution.of higher ionic strength than that used in step a) is contacted with the affinity chromatographic material such that said vitamin K dependent factors are eluted, then this fraction is either processed further to give a PPSB preparation, or these fractions containing the factors are pooled and then said fractions
c) are subjected to a second chromatographic step in the form of a heparin affinity chromatography,
wherein the source is undiluted plasma.

2. The process according to claim 1, wherein a virus inactivation step is performed between steps b) and c).

3. The process according to claim 2, wherein said virus inactivation is performed by treatment of the fractions containing said vitamin K dependent factors with non-ionic surfactants in the presence of dialkyl or trialkylphosphates with or without a heat treatment.

4. The process according to at least one of claims 1 to 3, wherein anion-exchange chromatography or a chromatography according to step a) of claim 1 is performed between steps b) and c).

5. The process according to at least one of claims 1 to 4, wherein another virus inactivation step is performed following step c) of claim 1.

6. The process according to claim 5, wherein said second virus inactivation step includes a heat treatment.

7. The process according to claim 5 or 6, wherein a third chromatographic purification operation is performed following said second virus inactivation step.

8. The process according to claim 7, wherein said third chromatographic purification operation is a heparin affinity chromatography, an anion-exchange chromatography, or a chromatography according to step a) of claim 1.

9. The process according to any of claims 1 to 8, wherein said source containing vitamin K dependent coagulation factors is blood plasma or cryopoor plasma.

10. The process according to claim 1, wherein said source containing vitamin K dependent coagulation factors is subjected to filtration prior to step a) of claim 1.

11. A PPSB fraction, obtainable by collecting the fractions following performance of step b) of claim 1.

12. Factor IX, obtainable by collecting the fractions following performance of step c) of claim 1 and the factor IX preparation is derived from blood plasma or cryopoor plasma.

13. A chromatographic material according to step a) of claim 1 obtainable by modification of tentacle material having a hydrophilic spacer bound on the base matrix, with 2-hydroxy aminopropyl groups.

## Patentansprüche

1. Verfahren zur Reinigung von Vitamin-K-abhängigen Gerinnungsfaktoren durch Affinitätschromatographie, wobei Fraktionen einer Quelle, die die Vitamin-K-abhängigen Faktoren enthält;
a) einer Chromatographie an einem Material, das mit 2-Hydroxyaminopropylgruppen modifiziert ist, die an Spacer mit mehr als 3 Kohlenstoffatomen gebunden sind, unterzogen werden, wobei die Quelle aus einer Lösung aufgetragen wird, deren Ionenstärke so gewählt ist, dass die Vitamin-K-abhängigen Gerinnungsfaktoren auf dem obigen Anionenaustauschmaterial adsorbiert werden, wobei gegebenenfalls mit einer geeigneten Pufferlösung gewaschen wird und dann, nach der Abtrennung des Eluats;
b) eine Pufferlösung mit größerer Ionenstärke als die in Schritt a) verwendete mit dem affinitätschromatographischen Material in Kontakt gebracht wird, so dass die Vitamin-K-abhängigen Faktoren eluiert werden, dann diese Fraktion entweder weiterverarbeitet wird, so dass man ein PPSB-Präparat erhält, oder diese Fraktionen, die die Faktoren enthalten, vereinigt werden und dann diese Fraktionen
c) einem zweiten Chromatographieschritt in Form einer Heparin-Affinitätschromatographie unterzogen werden;
wobei es sich bei der Quelle um unverdünntes Plasma handelt.

2. Verfahren gemäß Anspruch 1, wobei zwischen den Schritten b) und c) ein Virusinaktivierungsschritt durchgeführt wird.

3. Verfahren gemäß Anspruch 2, wobei die Virusinaktivierung durch Behandlung der Fraktionen, die die Vitamin-K-abhängigen Faktoren enthalten, mit nichtionischen Tensiden in Gegenwart von Dialkyl- oder Trialkylphosphaten mit oder ohne Wärmebehandlung durchgeführt wird.

4. Verfahren gemäß wenigstens einem der Ansprüche 1 bis 3, wobei zwischen den Schritten b) und c) eine Anionenaustauschchromatographie oder eine Chromatographie gemäß Schritt a) von Anspruch 1 durchgeführt wird.

5. Verfahren gemäß wenigstens einem der Ansprüche 1 bis 4, wobei nach dem Schritt c) von Anspruch 1 ein weiterer Virusinaktivierungsschritt durchgeführt wird.

6. Verfahren gemäß Anspruch 5, wobei der zweite Virusinaktivierungsschritt eine Wärmebehandlung beinhaltet.

7. Verfahren gemäß Anspruch 5 oder 6, wobei nach dem zweiten Virusinaktivierungsschritt eine dritte chromatographische Reinigungsoperation durchgeführt wird.

8. Verfahren gemäß Anspruch 7, wobei die dritte chromatographische Reinigungsoperation eine Heparin-Affinitätschromatographie, eine Anionenaustauschchromatographie oder eine Chromatographie gemäß Schritt a) von Anspruch 1 ist.

9. Verfahren gemäß einem der Ansprüche 1 bis 8, wobei es sich bei der Quelle, die die Vitamin-K-abhängigen Gerinnungsfaktoren enthält, um Blutplasma oder Cryopoor-Plasma handelt.

10. Verfahren gemäß Anspruch 1, wobei die Quelle, die die Vitamin-K-abhängigen Gerinnungsfaktoren enthält, vor Schritt a) von Anspruch 1 einer Filtration unterworfen wird.

11. PPSB-Fraktion, die erhältlich ist, indem man die Fraktionen nach der Durchführung von Schritt b) von Anspruch 1 auffängt.

12. Faktor IX, der erhältlich ist, indem man die Fraktionen nach der Durchführung von Schritt c) von Anspruch 1 auffängt, wobei das Faktor-IX-Präparat aus Blutplasma oder Cryopoor-Plasma stammt.

13. Chromatographisches Material gemäß Schritt a) von Anspruch 1, das durch Modifikation von Tentakelmaterial, das einen an die Grundmatrix gebundenen hydrophilen Spacer aufweist, mit 2-Hydroxyaminopropylgruppen erhältlich ist.

## Revendications

1. Procédé pour la purification des facteurs de coagulation dépendant de la vitamine K par chromatographie d'affinité, dans lequel des fractions d'une source contenant lesdits facteurs dépendant de la vitamine K sont :
a) soumises à une chromatographie sur un matériau modifié avec des groupes 2-hydroxyaminopropyle fixés à des segments intercalaires ayant plus de 3 atomes de carbone, ladite source étant appliquée à partir d'une solution dont la force ionique est choisie de telle sorte que lesdits facteurs de coagulation dépendant de la vitamine K soient adsorbés sur le matériau échangeur d'anions ci-dessus, le lavage est facultativement réalisé avec une solution tampon appropriée, puis après séparation de l'éluat,
b) une solution tampon ayant une force ionique supérieure à celle utilisée à l'étape a) est mise en contact avec le matériau pour chromatographie d'affinité de telle sorte que lesdits facteurs dépendant de la vitamine K soient élués, puis cette fraction est soit traitée davantage pour donner une préparation de PPSB, soit ces fractions contenant les facteurs sont regroupées et puis lesdites fractions
c) sont soumises à une seconde étape chromatographique sous la forme d'une chromatographie d'affinité pour l'héparine, dans laquelle la source est le plasma non dilué.

2. Procédé selon la revendication 1, dans lequel une étape d'inactivation virale est réalisée entre les étapes b) et c).

3. Procédé selon la revendication 2, dans lequel ladite inactivation virale est réalisée par traitement des fractions contenant lesdits facteurs dépendant de la vitamine K avec des tensioactifs non ioniques en présence de dialkyl- ou de trialkylphosphates avec ou sans traitement thermique.

4. Procédé selon au moins l'une des revendications 1 à 3, dans lequel une chromatographie échangeuse d'anions ou une chromatographie selon l'étape a) de la revendication 1 est réalisée entre les étapes b) et c).

5. Procédé selon au moins l'une des revendications 1 à 4, dans lequel une autre étape d'inactivation virale est réalisée à la suite de l'étape c) de la revendication 1.

6. Procédé selon la revendication 5, dans lequel ladite seconde étape d'inactivation virale comprend un traitement thermique.

7. Procédé selon la revendication 5 ou 6, dans lequel une troisième opération de purification chromatographique est réalisée à la suite de ladite seconde étape d'inactivation virale.

8. Procédé selon la revendication 7, dans lequel ladite troisième opération de purification chromatographique est une chromatographie d'affinité pour l'héparine, une chromatographie échangeuse d'anions ou une chromatographie selon l'étape a) de la revendication 1.

9. Procédé selon l'une quelconque des revendications 1 à 8, dans lequel ladite source contenant les facteurs de coagulation dépendant de la vitamine K est du plasma sanguin ou du plasma pauvre en cryoprécipité.

10. Procédé selon la revendication 1, dans lequel ladite source contenant les facteurs de coagulation dépendant de la vitamine K est soumise à une filtration avant l'étape a) de la revendication 1.

11. Fraction de PPSB, pouvant être obtenue en collectant les fractions suite à la réalisation de l'étape b) de la revendication 1.

12. Facteur IX, pouvant être obtenu en collectant les fractions suite à la réalisation de l'étape c) de la revendication 1 et la préparation du facteur IX est dérivée du plasma sanguin ou du plasma pauvre en cryoprécipité.

13. Matériau chromatographique selon l'étape a) de la revendication 1, pouvant être obtenu par modification d'un matériau tentaculaire (« tentacle material ») ayant un segment intercalaire hydrophile fixé sur la matrice de base, avec des groupes 2-hydroxyaminopropyle.
